(19) Europäisches Patentamt
European Patent Office
Office européen des brevets

(11) Publication number: **0 007 160**
**A1**

(12) # EUROPEAN PATENT APPLICATION

(21) Application number: 79300841.8

(22) Date of filing: 16.05.79

(51) Int. Cl.$^3$: **C 07 D 213/61**

(30) Priority: 19.06.78 US 916450

(43) Date of publication of application: 23.01.80 Bulletin 80/2

(84) Designated Contracting States: BE CH DE FR GB IT NL

(71) Applicant: THE DOW CHEMICAL COMPANY
2030 Abbott Road Post Office Box 1967
Midland Michigan 48640(US)

(72) Inventor: Dietsche, Thomas James
30, Roanoke Road, Berkeley
Alameda California(US)

(72) Inventor: Love, Jim
3378 Whitehaven Drive Walnut Creek
Contra Costa California(US)

(74) Representative: Allard, Susan Joyce et al,
BOULT, WADE & TENNANT 34 Cursitor Street
London EC4A 1PQ(GB)

(54) **Preparation of polychlorinated pyridines from 2,4-dichloro-6-(trichloromethyl)pyridine.**

(57) Pentachloropyridine and polychloro derivatives of 2-(trichloromethyl)pyridine are prepared by reacting 2,4-dichloro-6-(trichloromethyl)pyridine in the liquid state with chlorine at temperatures of at least 160°C in the presence of a Lewis acid type catalyst. The products are useful as herbicides, pesticides, or as chemical intermediates for the preparation of other products useful, for example, as herbicides or pesticides.

EP 0 007 160 A1

Croydon Printing Company Ltd.

# PREPARATION OF POLYCHLORINATED PYRIDINES FROM 2,4-DICHLORO-6-(TRICHLOROMETHYL)PYRIDINE

The present invention concerns the preparation of pentachloropyridine and polychloro pyridines such as 2,3,4-trichloro-, 2,4,5-trichloro- and 2,3,4,5-tetrachloro-6-(trichloromethyl)pyridine.

The chlorinated pyridine derivatives prepared by the process of the present invention are known compounds having been previously prepared by a number of processes. These compounds have uses such as herbicides, pesticides, etc., and are also employed as chemical intermediates in the preparation of other highly desired herbicide or pesticide products. Previous methods for preparing such compounds include those described in U.S. Patent Nos. 3,538,100 and 3,186,994. Thus, according to the '100 patent, pentachloropyridine (hereinafter referred to for convenience as "penta") has been prepared by chlorination of liquid 2,6-dichloropyridine at temperatures of at least about 180°C and in the presence of a metallic halide catalyst. Polychloropyridines, including penta, are also produced according to the '994 patent by chlorinating a polychloro(trichloromethyl)pyridine reactant in the

liquid state at a temperature of at least 160°C, preferably under irradiation with ultraviolet light.

According to the present invention, it has been found that chlorinated pyridine compounds selected from the group consisting of pentachloropyridine, 2,3,4-trichloro-, 2,4,5-trichloro- and 2,3,4,5-tetrachloro-6-(trichloromethyl)pyridine can be obtained in a process which comprises contacting liquid 2,4-dichloro-6-(trichloromethyl)pyridine with chlorine at temperatures of from about 160°C in the presence of a Lewis acid type catalyst. The reaction can be practiced to convert most or all of the 2,4-dichloro-6--(trichloromethyl)pyridine to pentachloropyridine, e.g., convert the trichloromethyl radical to a chloro radical with accompanying replacement of hydrogen on the ring, or to obtain optimum amounts of the polychloro derivatives of (trichloromethyl)pyridine. The process can also be carried out to provide mixtures of said products which can be separated from each other. The process is preferably carried out under anhydrous conditions. Use of the 2,4-dichloro- and 2,3,4-trichloro- and/or 2,4,5-trichloro-6-(trichloromethyl)-pyridine derivatives or mixtures thereof as starting materials also forms a part of the invention.

The process of the present invention is carried out by preferably using 2,4-dichloro-6-(trichloromethyl)pyridine as the starting material, although mixtures of the same with the trichloro derivatives, or one of the trichloro derivatives alone can be used as a starting reactant to prepare 2,3,4,5-tetrachloro--6-(trichloromethyl)pyridine and/or pentachloropyridine. The invention, in its broadest aspect, thus

comprises contacting a starting material of the formula:

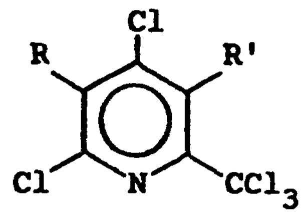

wherein R and R' are each independently chloro or H, with the proviso that at least one of R and R' is H, in the liquid phase with chlorine at a temperature of at least about 160°C and in the presence of a Lewis acid type catalyst. Preferably, a starting material wherein R and R' are both H is employed. In another embodiment, a starting material wherein R is chloro and R' is H is preferably employed. In still another embodiment, a starting material wherein R is H and R' is chloro is preferred. In a further embodiment, the starting material preferably comprises a mixture of the compounds encompassed by the above formula.

In carrying out the process of the present invention, gaseous chlorine is contacted with a liquid starting material at a temperature of at least about 160°C in the presence of a selected catalyst. Atmospheric or superatmospheric pressures can be employed.

Although an equimolar amount of the chlorine gas reactant may be employed, preferably from about 0.3 to about 10 excess molar proportions of chlorine per mole of starting material is employed. The continuous passage of excess chlorine gas through the reaction mixture serves not only to supply a large amount of reactant, but to sweep out any carbon tetrachloride or hydrogen chloride by-products. The most suitable rate

at which the chlorine gas is fed will vary with the reaction temperature, pressure, reaction mixture volume, end-product desired, etc. An excess amount of from about 0.3 to about 5.0 moles of chlorine per hour is usually employed per mole of starting material.

Representative catalysts include, for example, Lewis acid type catalysts such as metals or metallic halides capable of being converted to covalent metallic chlorides under the conditions of the chlorination reaction of the present invention. Metals themselves such as iron, zinc, aluminum, and the like can be employed, preferably in the powdered form. Representative covalent metallic chlorides or halides which can be converted to the chloride form include those such as ferric chloride, ferric bromide, aluminum chloride, aluminum bromide, antimony pentachloride, molybdenum pentachloride, tungsten hexachloride, boron trifluoride, titanic chloride, nickel chloride and similar materials. As will be understood by those skilled in the art, no equivalency in activity or operability of the catalyst materials is to be inferred. While certain catalysts have been found to provide good results over a short reaction period, for example, at atmospheric pressure, others which may be operable may require long reaction time periods. Further, certain catalysts may be superior when employed at elevated temperatures and/or pressures. The degree of catalytic activity may also vary depending upon the particular product which is to be produced. In any event, those skilled in the art can, by routine experimentation according to the teachings of the specification and examples herein, readily determine the optimum catalyst and amount thereof required for any particular

product to be made or for any particular set of pressure, temperature or time conditions desired. Catalysts bonded to inert supports or the use of co-catalysts are also contemplated for use in the present invention. Catalysts preferred for use in the present invention include halides of ruthenium, tantalum, tungsten, molybdenum, niobium, aluminum, zinc, and iron. Highly preferred catalysts for use in the present invention include the ferric and aluminum halides, and iron and aluminum metals. A preferred catalyst is ferric chloride. The catalysts are usually employed in an amount ranging from about 1 to about 20 mole percent based on the amount of starting material. Preferably, a catalyst concentration of from about 1.0 to about 10 mole percent is employed.

In a preferred method for carrying out the process of the present invention, 2,4-dichloro-6-(trichloromethyl)pyridine starting material in liquid, e.g., molten form is added to a reactor previously heated to at least about 100°C and the reactor purged with nitrogen. A catalyst is then added, and chlorine flow commenced at a sufficient rate to pressure the reactor, usually to about 15 psig (1.05 $kg/cm^2$ gauge) or more. The temperature of the reactor is then slowly increased to at least about 160°C and the reaction maintained until sufficient amounts of the desired pyridine compounds are obtained. Liquid samples from the reactor and vent gases are periodically taken and analyzed by known methods to monitor the course of the reaction. The reaction is terminated by stopping the heating of the reactor and the flow of chlorine thereto and allowing the reactor pressure to drop to atmospheric. Distillation of the reaction product obtained can then be carried out to obtain the desirable products

therefrom and the still bottoms can be recovered and re-used.

The reaction process starting with 2,4-dichloro-6-(trichloromethyl)pyridine is generally illustrated as follows:

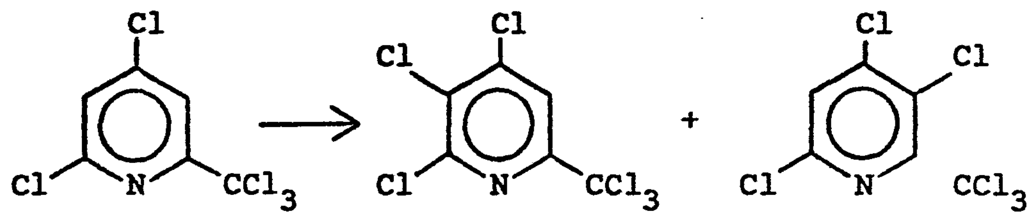

(I) (II) (III)

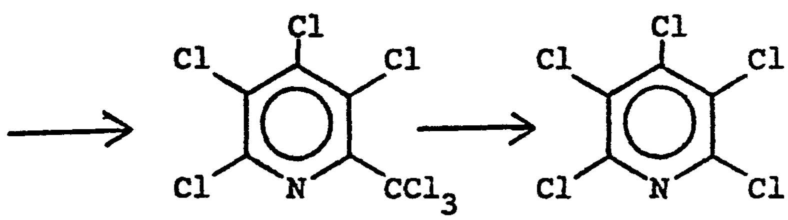

(IV) (V)

During the initial stage of the reaction sequence the 2,4-dichloro-6-(trichloromethyl)pyridine starting material (I) is largely converted to the 2,3,4-trichloro-6-(trichloromethyl)pyridine (II), with lesser amounts of the 2,4,5-trichloro-6-(trichloromethyl)pyridine (III) and some small amounts of 2,3,4,5-tetrachloro-6-(trichloromethyl)pyridine (IV) and pentachloropyridine (V) being formed. As the reaction continues, products (II) and (III) reach optimum amounts and then begin to decrease as the levels of (IV) and (V) continue to increase. If the reaction sequence is continued for a long enough period, a product comprising (V) as the chief or primary product can be obtained. The course of the reaction can, of course, be monitored and the

reaction sequence stopped whenever optimum amounts of one or more of the desired products have been obtained.

In a preferred embodiment, the starting material is (I) and the process is selectively practiced to produce a mixture of chlorinated pyridine compounds comprising product (II) as the primary or chief component of the mixture. In another embodiment wherein (I) is the starting material, the process is selectively practiced to obtain (IV) as the primary or chief product contained in the mixture. In still another embodiment using (I) as the starting material, the process is selectively practiced to obtain (V) as the primary or chief product of the mixture. In a further preferred embodiment, (II) is the starting material.

While the process of the present invention can be conducted at atmospheric pressure, it is usually advantageous to employ at least slightly increased pressures. Thus pressures of from about 15 psig to about 220 psig (1.05-15.4 kg/cm$^2$ gauge) are preferably employed. In all embodiments of the invention, the only constraint placed upon the superatmospheric pressures employed is one of economics and pressures in excess of 220 psig (15.4 kg/cm$^2$ gauge) may be employed; however, those skilled in the art will recognize that the cost factor for pressure equipment to allow operation above 200-220 psig (14-15.4 kg/cm$^2$ gauge) is greatly increased and that the additional cost may exceed any benefits obtained. In a preferred embodiment, the process is carried out at temperatures of from about 160 to about 220°C and pressures of from atmospheric to about 220 psig (15.4 kg/cm$^2$ gauge), and in

the presence of from about 1 to about 10 mole percent of a catalyst based on the starting material. In another preferred embodiment, the process of the present invention is carried out at temperatures of from about 190 to about 220°C, at pressures of from about 190 to about 220 psig (13.3-15.4 kg/$cm^2$ gauge) and in the presence of from about 1 to about 10 mole percent catalyst.

The 2,4-dichloro-6-(trichloromethyl)pyridine starting material is known and can be obtained from methods known in the art. The products (II)-(V) likewise are known in the art as are their physical properties.

The following examples illustrate the process of the present invention but are not to be construed as limiting the same. The analysis of the reaction mixtures was accomplished by vapor phase chromatography. The product distribution in all tables is in terms of weight percent.

Example 1

A chlorination reactor comprising a 300 ml flask fitted with a sparge tube connected via a rotometer and needle valve to a chlorine source and a condensor connected to a caustic scrubber was charged with a melt of a 2,4-dichloro-6-(trichloromethyl)pyridine (98.2%) starting material. The reaction mixture was then heated at about 200°C at atmospheric pressure with stirring and chlorine was sparged into the solution at the rate of about 0.1 mole/hr. Samples were removed via a sampling port at various intervals. The reaction was conducted for a period of about 8 hours, with samples being taken at the second, fourth and eighth hour

intervals. Analysis of the samples indicated the starting material remained unchanged. At the end of 8 hours, 5 mole percent of Fe catalyst was charged to the reactor and the reaction sequence continued with samples being taken at various intervals. The results of such operation are set forth in the following Table A:

TABLE A

Temperature: 200°C    Pressure: Atmospheric
Catalyst: 5 mole % Fe

| Sample | Time (Hrs.) | *I | II | III | IV |
|---|---|---|---|---|---|
| 1 | 0 | 98.2 | -- | -- | -- |
| 2 | 1 | 75.6 | 15.0 | 7.7 | 0.2 |
| 3 | 2 | 59.0 | 25.7 | 13.1 | 0.6 |
| 4 | 3.5 | 31.6 | 42.7 | 21.4 | 2.8 |
| 5 | 6 | 8.3 | 48.9 | 21.5 | 20.2 |
| 6 | 7 | 8.1 | 43.3 | 17.3 | 30.3 |
| 7 | 8 | 9.2 | 34.8 | 12.3 | 42.9 |
| 8 | 9 | 11.9 | 24.5 | 7.4 | 53.8 |
| 9 | 10 | 12.9 | 17.1 | 4.2 | 62.8 |
| 10 | 11 | 14.0 | 11.4 | 2.7 | 68.6 |
| 11 | 12 | 15.8 | 5.8 | 1.7 | 72.8 |
| 12 | 14 | 20.0 | 0.4 | 1.1 | 74.8 |
| 13 | 16 | 26.1 | 0.1 | 0.2 | 68.8 |

*G.C. peak for I coincides with peak for Product V, which begins to appear at about Sample No. 8.

The above example demonstrates the need for a catalyst and the effect thereof on the production of products (II)-(V) hereof.

Example 2

In other operations utilizing the procedures of Example 1, Product II was utilized as a starting material to prepare products (IV) and (V). The reaction was conducted for 8 hours without a catalyst, and then for 8 hours with a catalyst added. The results are set forth in Table B:

TABLE B

Temperature: 200°C Pressure: Atmospheric

| Sample | Time (Hrs.) | II | IV | V |
|---|---|---|---|---|
| 1 | 0 | 98.9 | -- | 0.1 |
| 2 | 1 | 98.7 | -- | 0.2 |
| 3 | 4 | 98.5 | -- | 0.1 |
| 4 | *8 | 98.5 | -- | 0.1 |
| **5 | 1.25 | 70.9 | 26.2 | 1.1 |
| 6 | 2 | 57.0 | 39.7 | 2.0 |
| 7 | 3 | 36.1 | 59.1 | 3.1 |
| 8 | 4 | 20.9 | 72.2 | 4.8 |
| 9 | 6 | 0.3 | 86.4 | 10.8 |
| 10 | 8 | -- | 76.0 | 21.0 |

*5 mole % Fe added after first 8 hours.

**Time in Runs 5-10 is hours from point of catalyst addition.

The preceding Example 2 again demonstrates the need for a catalyst as well as the use of product (II) as a starting material to prepare products (IV) and (V). From the data set forth in Examples 1-2, it will be apparent to one skilled in the art that the reaction can be terminated whenever optimum amounts of

one or more of the products is obtained and such product or products can thereafter be recovered by distillation, selective crystallization, etc. The process may be conducted in a batchwise manner, continuous, or a cyclic batch wherein the desired products are continuously or periodically removed leaving a quantity of the reaction mass in the reactor and adding makeup starting material and catalyst, or by distilling the product as it is removed leaving the catalyst in a distillation residue which can be recycled with makeup catalyst and reactant to the reactor. The continuous or cyclic processes can also be carried in a series of reactors if so desired.

The best mode for carrying out the most preferred embodiment of the invention presently known to the inventors is believed to be the chlorination of 2,4-dichloro-6-(trichloromethyl)pyridine at a temperature of from about 190 to about 220°C in the presence of about 5 mole percent $FeCl_3$ catalyst at atmospheric pressure, although it is believed use of superatmospheric pressures may be advantageous in increasing the reaction rate.

CLAIMS

1. A process for preparing chlorinated pyridine compounds which comprises contacting in the liquid phase a starting material of the general formula:

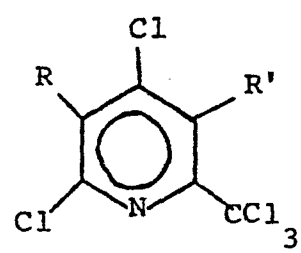

wherein R and R' each independently represent a chlorine atom or a hydrogen atom, with the proviso that at least one of R and R' is a hydrogen atom, with chlorine at a temperature of at least 160°C in the presence of a Lewis acid type catalyst, and thereafter recovering the chlorinated pyridine compounds.

2. A process as claimed in claim 1 wherein R and R' are both hydrogen atoms and the chlorinated pyridine compounds are selected from 2,3,4-trichloro-, 2,4,5-trichloro- and 2,3,4,5-tetrachloro-6-(trichloromethyl)pyridine and pentachloropyridine.

3. A process as claimed in claim 1 wherein in the starting material R is a chlorine atom and R' is a hydrogen atom.

4. A process as claimed in claim 1 wherein in the starting material R is a hydrogen atom and R' is a chlorine atom.

5. A process as claimed in any one of the preceding claims wherein the process is carried out at a temperature in the range of from 160 to 220°C and at pressures in the range of from atmospheric to 220 psig (15.4 $kg/cm^2$ gauge).

6. A process as claimed in claim 5 wherein the temperature is in the range of from 190 to 220°C, and the pressures is in the range of from 190 to 220 psig (13.3-15.4 $kg/cm^2$ gauge).

7. A process as claimed in any one of the preceding claims which is carried out in the presence of an excess of from 0.3 to 10 molar proportions of chlorine per mole of starting material.

8. A process as claimed in any one of the preceding claims which is carried out in the presence of from 1 to 10 mole percent catalyst based on the starting material.

9. A process as claimed in any one of the preceding claims wherein the catalyst is an iron or aluminium metal or halide thereof.

10. A process as claimed in any one of the preceding claims wherein the catalyst is ferric chloride.

SJA/EA 056

European Patent Office

# EUROPEAN SEARCH REPORT

Application number

EP 79 30 0841

## DOCUMENTS CONSIDERED TO BE RELEVANT

| Category | Citation of document with indication, where appropriate, of relevant passages | Relevant to claim |
|---|---|---|
| | FR - A - 1 347 180 (DOW)<br>* pages 1-10 *<br>--- | 1-4,9,<br>10 |
| | US - A - 3 732 230 (T. BREWER)<br>* claims 1-5 *<br>--- | 1,5,6,<br>7,8 |
| D | US - A - 3 538 100 (E. SMITH)<br>* whole patent *<br>--- | 1,5-10 |
| D | US - A - 3 186 994 (H. JOHNSTON)<br>* claims 1-4 *<br>--------- | 1-4 |

CLASSIFICATION OF THE APPLICATION (Int. Cl.[3])

C 07 D 213/61

TECHNICAL FIELDS SEARCHED (Int.Cl.[3])

C 07 D 213/61

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 13-07-1979 | VERHULST |